# EUROPEAN PATENT APPLICATION

(11) **EP 3 425 403 A1**
(43) Date of publication of application: **09.01.2019**
(21) Application number: 17179649.3
(22) Date of filing: 04.07.2017
(51) Int. Cl.: G01N 33/68

(54) **METABOLITE MARKER COMBINATIONS FOR PREDICTION OF CORONARY HEART DISEASE**

(71) Applicant: Universitätsklinikum Hamburg-Eppendorf, 20246 Hamburg (DE)
(72) Inventor: ZELLER, Tanja, 22307 Hamburg (DE); BLANKENBERG, Stefan, 20251 Hamburg (DE); BÖHRNIGEN, Daniela, 21255 Tostedt (DE)
(74) Representative: Wünsche, Annelie

(57) **Abstract**

The present invention relates to the use of a maker combination comprising at least two metabolite markers for assessing the risk of coronary heart disease in a subject, wherein one metabolite marker is asparagine and wherein at least one further metabolite marker is selected from the group consisting of glutamine, glutamate, acetylcarnitine and phosphoglycerides. The invention further refers to the method of predicting the risk of coronary heart disease in apparently health subjects.

## Description

### FIELD OF THE INVENTION

The present invention relates to the use of a metabolite maker combination comprising at least two metabolite markers for assessing the risk of coronary heart disease in a subject, wherein one metabolite marker is asparagine and wherein at least one further metabolite marker is selected from the group consisting of glutamine, glutamate, acetylcarnitine and phosphoglycerides. The invention further refers to the method of predicting the risk of coronary heart disease in apparently healthy subjects.

### BACKGROUND OF THE INVENTION

Despite significant advances in treatment, cardiovascular disease remains the leading cause of death world-wide. In particular, coronary heart disease (CHD) morbidity and mortality pose a major public health burden worldwide, since deaths from cardiovascular disease are still increasing. Coronary heart disease (CHD) has a complex and heterogenic etiology involving numerous environmental and genetic factors. CHD has a complex and heterogenic etiology involving numerous environmental and genetic factors.

Tools for the prediction of the cardiovascular events are incomplete and a considerable number of patients at risk cannot be identified on the basis of traditional risk factors alone. In order to improve risk estimation and to improve therapy decision making and guidance there is a need for novel methods and novel marker combinations to better predict the risk of cardiovascular events, in particular coronary heart disease.

CHD risk assessment might be improved by large-scale, high-throughput, and sex-specific analyses on a molecular level.

### OBJECTIVES AND SUMMARY OF THE INVENTION

To achieve this object the invention is directed to the use of a metabolite marker combination comprising at least two metabolite markers for assessing the risk of coronary heart disease in a subject, wherein one marker is asparagine and wherein at least one further metabolite marker is selected from the group consisting of glutamine, glutamate, acetylcarnitine and phosphoglyceride.

The combination of asparagine with at least one further metabolite marker selected from the group consisting of glutamine, glutamate, acetylcarnitine and phosphoglycerides is capable to predict the risk of coronary heart disease.

Preferable, the phosphoglyceride is selected from the group consisting of PC aa C40:2, PC ae C44:5, PC aa C43:3 and PC ae C38:1. More preferable the phosphoglyceride is PC aa C40:2 or PC ae C44:5.

In some embodiments, the metabolite marker combination comprising at least two metabolite markers is selected from the group consisting of:
- asparagine and glutamine,
- asparagine and acetylcarnitine,
- asparagine, acetylcarnitine and PC ae C44:5,
- asparagine and PC aa C40:2,
- asparagine and PC ae C44:5,
- asparagine, glutamine and glutamate,
- asparagine, glutamine and PC aa C40:2,
- asparagine, glutamine and PC ae C44:5,
- asparagine, glutamate and PC aa C40:2,
- asparagine, glutamate and PC ae C44:5,
- asparagine, glutamate, PC aa C34:3, and PC ae C44:5,
- asparagine, PC aa C40:2 and PC ae C44:5,
- asparagine, glutamine, glutamate and PC aa C40:2,
- asparagine, glutamine, glutamate and PC ae C44:5, and
- asparagine, glutamine, PC aa C40:2 and PC ae C44:5.

In specific embodiments, the metabolite marker combination comprising at least two metabolite markers is selected from the group consisting of:
- asparagine and glutamine,
- asparagine and acetylcarnitine,
- asparagine and PC ae C44:5, and
- asparagine, acetylcarnitine and PC ae C44:5.

Typically, the subject is male or female. In some embodiments, the subject is female.

In some embodiments, the metabolite marker combination comprising at least two metabolite markers is selected from the group consisting of:
- asparagine and glutamine, and
- asparagine, glutamate, PC aa C34:3 and PC ae C44:5.

In specific embodiments, the metabolite marker combination comprising at least two metabolite markers is selected from the group consisting of:
- asparagine and glutamine, and
- asparagine, glutamate, PC aa C34:3 and PC ae C44:5,
wherein the subject is male or female.

In some embodiments, the metabolite marker combination is selected from the group consisting of:
- asparagine and acetylcarnitine, and
- asparagine, acetylcarnitine and PC ae C44:5.

In specific embodiments, the metabolite marker combination is selected from the group consisting of:
- asparagine and acetylcarnitine, and
- asparagine, acetylcarnitine and PC ae C44:5,
wherein the subject is female.

In some embodiments, the subject did not suffer from a cardiovascular event. In other embodiments, the subject did suffer from a cardiovascular event.

Typically, the sample is a body fluid of the subject, such as a blood sample (serum and plasma) or urine sample of the subject. In preferred embodiments, the sample is a blood sample (serum and plasma) of the subject.

The level of the maker may be detected by mass spectrometry or nuclear magnetic resonance spectroscopy.

Another aspect of the invention refers to a method for assessing the risk of coronary heart disease in a subject comprising the following steps:
a) detecting the level of at least two makers in a sample from the subject wherein the at least two markers are as defined herein;
b) assessing the risk of coronary heart disease in the subject based on the level of the at least two makers.

Typically, step b) comprises comparing the level of the metabolite markers in the sample to a standard, wherein the level of the metabolite markers in the sample of the subject is indicative for the risk of coronary heart disease in the subject.

The invention also refers to a method for assessing the risk of coronary heart disease in a subject comprising the following steps:
a) providing the sample to a laboratory for detecting the level of at least two makers in a sample, wherein the at least two metabolite markers are as defined herein;
b) assessing the risk of coronary heart disease in the subject based on the level of the at least two makers based on the report provided from the laboratory indicating the level of the at least two metabolite markers in the sample.

### FIGURE LEGENDS

Figure 1: **Forest plot of Hazard Ratios and p-values of all significant metabolites associated with incident CHD.** For each significant metabolite the Hazard Ratio (per SD) and its corresponding 95% confidence interval is visualized as a forest plot for women, men, and the entire cohort (overall). Additionally, corresponding p-values after correction for multiple testing (Bonferroni) are presented. Significant p-values are highlighted in red.
Figure 2: **Heatmap of pairwise correlation coefficients between significant metabolites in CHD.** Pairwise correlation (Pearson correlation coefficient) between the metabolites significantly associated with incident CHD in women.
Figure 3: **Heatmap of pairwise correlation coefficients between significant metabolites in CHD.** Pairwise correlation (Pearson correlation coefficient) between the metabolites significantly associated with incident CHD in the entire case-cohort.
Figure 4: **Survival curves of metabolite signatures in incident CHD in women and men for 5 year survival.** In women, the metabolite signature C2 + Asn + PC aa 40:5 shows the strongest improvement of discrimination, while all metabolites at high levels (>mean concentration) are associated with decreased risk of incident CHD (Hazard Ratios < 1).

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

To achieve this object the invention is directed to the use of a metabolite marker combination comprising at least two metabolite markers for assessing the risk of coronary heart disease in a subject, wherein one metabolite marker is asparagine and wherein at least one further metabolite marker is selected from the group consisting of glutamine, glutamate, acetylcarnitine and phosphoglycerides.

The term "coronary heart disease" refers to conditions in which plaque builds up inside the coronary arteries, which supply oxygen-rich blood to your heart muscle.

Use of a metabolite marker is understood as measuring the concentration of the metabolite marker in the sample of a patient.

Typically, the concentration of the metabolite marker in the sample of the patient is compared to a "standard value". The "standard value" may be the mean concentration of a cohort of healthy patients.

The term "assessment of risk of coronary heart disease" refers to the prediction indicating that an apparently healthy subject has a higher chance of developing a coronary heart disease in the future compared to a control subject, e.g. that the subject is 10%, 20%, 30%, 40% 50%, 60%, 70%, 100%, 200%, 500%, 1000% or more likely to develop a coronary heart disease in the future than a control subject.

At least two metabolite markers also encompasses, at least 3, at least 4, at least 5, at least 6 or more metabolite markers. Preferably, less than 100, more preferably less than 20, even more preferably less than 10, still more preferably 8, most preferably less than 6 metabolite markers are used.

The subject may be a mammal. Typically, the subject is a human. The subject may be already identified as having a risk of developing coronary heart disease. Then the invention is used to confirm or to further analyze the risk of developing coronary heart disease. Alternatively, the patient's risk of developing coronary heart disease may be unknown.

The term "metabolite marker" refers to any substance produced or used during all the physical and chemical processes within the body that create and use energy, such as: digesting food and nutrients, eliminating waste through urine and faeces, breathing, circulating blood, and regulating temperature. In particular, the term refers to compounds like amino acids, biogenic amines, acylcarnitines, glycerophospholipids, sphingolipids and sugars. More particular, the term relates to acetylcarnitine, phosphoglycerides and amino acids, such as glutamine, glutamate.

If the value of the "protective metabolite" is decreased, i.e. is lower than a predetermined value than an increased risk for coronary heart disease may exist. "Protective metabolites" are metabolites showing a Hazard Ratio < 1. Therefore, asparagine, glutamine and acetylcarnitine are considered as protective metabolites.

In other words, the concentration of asparagine, glutamine and/or acetylcarnitine below a predetermined value indicates that an increased risk of coronary heart disease exists.

On the contrary if the value of a "risk metabolite" is increased, i.e. is higher than a predetermined value than an increased risk of coronary heart disease exists. "Risk metabolites" are metabolites that show a Hazard Ration of > 1. Therefore, glutamate, PC aa C40:2, PC ae C44:5, PC aa C34:3 and PC ae C38:1 are considered as "risk metabolites".

In other words, the concentration of glutamate, PC aa C40:2, PC ae C44:5, PC aa C34:3 and/or PC ae C38:1 above a predetermined value indicates that an increased risk of coronary heart disease exists.

The Hazard ratio may be determined by Cox proportional Hazard models (David Cox: Regression models and life tables. Journal of the Royal Statistical Society B, 34 (1972), page 187 - 220). The model may be adjusted for the covariates such as age, sex, blood pressure, HDL and LDL cholesterol, body-mass-index (BMI), prevalent diabetes, the different cohort studies and medication (antihypertensive treatment, lipid lowering treatment).

The predetermined value may be determined based on a large population study. Alternatively, the predetermined value may be determined based on one or more control subjects.

The mean values can for example be determined based on the BiomarCaRE case cohort study.

Accordingly, if the concentration of asparagine in a patient is below 5.0 to 8.5 µM, preferably 0.5 to 6.9 µM, more preferably about 6.4 to 6.5 µM then the risk of developing heart disease is increased.

If the concentration of glutamine in a patient is below 0.3 to 2.5 µM, preferably 0.4 to 1.9 µM, such as about 1.3 µM then the risk of developing heart disease is increased.

Accordingly, if the concentration of acetylcarnitine in a patient is below 0.5 to 3 µM, preferably 0.8 to 2.3 µM, such as about 1.6 µM then the risk of developing heart disease is increased.

If the concentration of glutamate is above 0.5 to 2.5 µM, preferably 1 to 2.1 µM, such as about 1.5 µM then the risk of developing heart disease is increased.

If the concentration of PC aa C40:2 is above 0.01 to 1 µM, preferably 0.03 to 0.8 µM, such as about 0.3 µM then the risk of developing heart disease is increased.

If the concentration of PC ae C44:5 is above 2.0 to 4.5 µM, preferably 2.4 to 4 µM, such as about 3.3 µM then the risk of developing heart disease is increased.

If the concentration of PC aa C34:3 is above 3 to 5 µM, preferably 3.5 to 4.4 µM, such as about 3.9 µM then the risk of developing heart disease is increased.

If the concentration of PC ae C38:1 is above 1 to 4 µM, preferably 1.1 to 3.6 µM, such as about 2.7 to 2.8 µM then the risk of developing heart disease is increased.

Preferably, the phosphoglyceride is phosphatidylcholine. More preferable, the phosphoglyceride is selected from the group consisting of PC aa C40:2, PC ae C44:5, PC aa C43:3 and PC ae C38:1. Even more preferable the phosphoglyceride is PC aa C40:2 or PC ae C44:5.

"PC aa C40:2" denotes a phosphatidylcholine having 40 C atoms and 2 double bonds in which the side chains are connected by an alkyl-alkyl bond.

"PC ae C44:5" denotes a phosphatidylcholine having 44 C atoms and 5 double bonds, which the side chains are connected by an alkyl-ether bond.

"PC ae C38:1" denotes a phosphatidylcholine having 39 C atoms and 1 double bond, which the side chains are connected by an alkyl-ether bond.

"PC aa C34:3" denotes a phosphatidylcholine having 34 C atoms and 3 double bonds which the side chains are connected by an alkyl-alkyl bond.

In specific embodiments, the metabolite marker combination comprising at least two metabolite markers is selected from the group consisting of:
- asparagine and glutamine, and
- asparagine, glutamate, PC aa C34:3 and PC ae C44:5,
wherein the subject is male or female.

In some embodiments, the metabolite marker combination comprising at least two metabolite markers is selected from the group consisting of:
- asparagine and glutamine,
- asparagine and acetylcarnitine,
- asparagine, acetylcarnitine and PC ae C44:5,
- asparagine and PC aa C40:2,
- asparagine and PC ae C44:5,
- asparagine, glutamine and glutamate,
- asparagine, glutamine and PC aa C40:2,
- asparagine, glutamine and PC ae C44:5,
- asparagine, glutamate and PC aa C40:2,
- asparagine, glutamate and PC ae C44:5,
- asparagine, PC aa C40:2 and PC ae C44:5,
- asparagine, glutamine, glutamate and PC aa C40:2,
- asparagine, glutamine, glutamate and PC ae C44:5,
- asparagine, glutamine, PC aa C40:2 and PC ae C44:5,
- glutamine and PC aa C40:2,
- asparagine and glutamate,
- acetylcarnitine amd PC ae C44:5,
- asparagine and PC ae C44:5,
- glutamine and PC ae C44:5,
- asparagine, acetylcarnitine, glutamine and glutamate,
- asparagine, acetylcarnitine, glutamine and PC ae C38:1,
- asparagine, acetylcarnitine, glutamate and PC ae C38:1,
- asparagine, acetylcarnitine, glutamate and PC ae C44:5,
- asparagine, glutamate, PC aa C34:3 and PC ae C44:5,
- asparagine, acetylcarnitine, PC ae C38:1 and PC ae C44:5,
- asparagine, glutamine, PC ae C38:1 and PC ae C44:5,
- asparagine, glutamate, PC ae C38:1 and PC ae C44:5,
- asparagine, acetylcarnitine, glutamine, glutamate and PC ae C38:1,
- asparagine, acetylcarnitine, glutamine, glutamate and PC ae C44:5,
- asparagine, acetylcarnitine, glutamine, PC ae C44:5 and PC ae C38:1,
- asparagine, acetylcarnitine, glutamate, PC ae C44:5 and PC ae C38:1,
- asparagine, glutamine, glutamate, PC ae C44:5 and PC ae C38:1,
- asparagine, acetylcarnitine, glutamine, glutamate, PC ae C44:5 and PC ae C38:1.

In preferred embodiments, the metabolite marker combination comprising at least two metabolite markers is selected from the group consisting of:
- asparagine and glutamine,
- asparagine and acetylcarnitine,
- asparagine, acetylcarnitine and PC ae C44:5,
- asparagine and PC aa C40:2,
- asparagine and PC ae C44:5,
- asparagine, glutamine and glutamate,
- asparagine, glutamine and PC aa C40:2,
- asparagine, glutamine and PC ae C44:5,
- asparagine, glutamate and PC aa C40:2,
- asparagine, glutamate and PC ae C44:5,
- asparagine, PC aa C40:2 and PC ae C44:5,
- asparagine, glutamine, glutamate and PC aa C40:2,
- asparagine, glutamine, glutamate and PC ae C44:5,
- asparagine, glutamine, PC aa C40:2 and PC ae C44:5, and
- asparagine, glutamate, PC aa C34:3 and PC ae C44:5.

The skilled person understands that at least the metabolite marker mentioned in one combination above are used for assessing the risk of coronary heart disease. It is understood that the specific metabolite marker combination with or without additional metabolite markers is covered. In one embodiment, the specific metabolite marker combination consists of the metabolite markers that are explicitly described, i.e. does not include additional metabolite markers than the one explicitly mentioned.

Accordingly, in specific embodiments the metabolite marker combination consists of the metabolite marker combination selected from the group consisting of:
- asparagine and glutamine,
- asparagine and acetylcarnitine,
- asparagine, acetylcarnitine and PC ae C44:5,
- asparagine and PC aa C40:2,
- asparagine and PC ae C44:5,
- asparagine, glutamine and glutamate,
- asparagine, glutamine and PC aa C40:2,
- asparagine, glutamine and PC ae C44:5,
- asparagine, glutamate and PC aa C40:2,
- asparagine, glutamate and PC ae C44:5,
- asparagine, PC aa C40:2 and PC ae C44:5,
- asparagine, glutamine, glutamate and PC aa C40:2,
- asparagine, glutamine, glutamate and PC ae C44:5,
- asparagine, glutamine, PC aa C40:2 and PC ae C44:5,
- glutamine and PC aa C40:2,
- asparagine and glutamate,
- acetylcarnitine and PC ae C44:5,
- asparagine and PC ae C44:5,
- glutamine and PC ae C44:5,
- asparagine, acetylcarnitine, glutamine and glutamate,
- asparagine, acetylcarnitine, glutamine and PC ae C38:1,
- asparagine, acetylcarnitine, glutamate and PC ae C38:1,
- asparagine, acetylcarnitine, glutamate and PC ae C44:5,
- asparagine, glutamate, PC aa C34:3 and PC ae C44:5,
- asparagine, acetylcarnitine, PC ae C38:1 and PC ae C44:5,
- asparagine, glutamine, PC ae C38:1 and PC ae C44:5,
- asparagine, glutamate, PC ae C38:1 and PC ae C44:5,
- asparagine, acetylcarnitine, glutamine, glutamate and PC ae C38:1,
- asparagine, acetylcarnitine, glutamine, glutamate and PC ae C44:5,
- asparagine, acetylcarnitine, glutamine, PC ae C44:5 and PC ae C38:1,
- asparagine, acetylcarnitine, glutamate, PC ae C44:5 and PC ae C38:1,
- asparagine, glutamine, glutamate, PC ae C44:5 and PC ae C38:1, and
- asparagine, acetylcarnitine, glutamine, glutamate, PC ae C44:5 and PC ae C38:1.

In preferred embodiments, the metabolite marker combination consists of the metabolite marker combination selected from the group consisting of:
- asparagine and glutamine,
- asparagine and acetylcarnitine,
- asparagine, acetylcarnitine and PC ae C44:5,
- asparagine and PC aa C40:2,
- asparagine and PC ae C44:5,
- asparagine, glutamine and glutamate,
- asparagine, glutamine and PC aa C40:2,
- asparagine, glutamine and PC ae C44:5,
- asparagine, glutamate and PC aa C40:2,
- asparagine, glutamate and PC ae C44:5,
- asparagine, PC aa C40:2 and PC ae C44:5,
- asparagine, glutamine, glutamate and PC aa C40:2,
- asparagine, glutamine, glutamate and PC ae C44:5,
- asparagine, glutamine, PC aa C40:2 and PC ae C44:5, and
- asparagine, glutamate, PC aa C34:3 and PC ae C44:5.

In specific embodiments the metabolite marker combination comprises at least two metabolite markers is selected from the group consisting of:
- asparagine and glutamine,
- asparagine and acetylcarnitine,
- asparagine and PC ae C44:5, and
- asparagine, acetylcarnitine and PC ae C44:5.

Typically, the subject is male or female. In some embodiments the subject is female.

In some embodiments, the metabolite marker combination comprising at least two metabolite markers is selected from the group consisting of:
- asparagine and glutamine, and
- asparagine, glutamate, PC aa C34:3 and PC ae C44:5.

In specific embodiments the metabolite marker combination comprising at least two metabolite markers is selected from the group consisting of:
- asparagine and glutamine, and
- asparagine, glutamate, PC aa C34:3 and PC ae C44:5,
wherein the subject is male or female.

In some embodiments, the metabolite marker combination is selected from the group consisting of:
- asparagine and acetylcarnitine, and
- asparagine, acetylcarnitine and PC ae C44:5.

In specific embodiments, the metabolite marker combination is selected from the group consisting of:
- asparagine and acetylcarnitine, and
- asparagine, acetylcarnitine and PC ae C44:5,
wherein the subject is female.

In some embodiments, the subject did not suffer from a cardiovascular event. In other embodiments the subject did suffer from a cardiovascular event.

The term "cardiovascular event" refers to cardiovascular disease symptoms like stroke or myocardial infarction.

Typically, the sample is a body fluid of the subject, such as a blood sample or urine sample of the subject. In preferred embodiments, the sample is a blood sample of the subject. Typically, the blood sample is serum or plasma.

The level of the maker may be detected by mass spectrometry or nuclear magnetic resonance spectroscopy.

Another aspect of the invention refers to a method for assessing the risk of coronary heart disease in a subject comprising the following steps:
a) detecting the level of at least two makers in a sample from the subject wherein the at least two metabolite markers are as defined herein;
b) assessing the risk of coronary heart disease in the subject based on the level of the at least two makers.

Typically, step b) comprises comparing the level of the metabolite markers in the sample to a standard, wherein the level of the metabolite markers in the sample of the subject is indicative for the risk of coronary heart disease in the subject.

The invention also refers to a method for assessing the risk of coronary heart disease in a subject comprising the following steps:
a) providing the sample to a laboratory for detecting the level of at least two makers in a sample, wherein the at least two metabolite markers are as defined herein;
b) assessing the risk of coronary heart disease in the subject based on the level of the at least two makers based on the report provided from the laboratory indicating the level of the at least two metabolite markers in the sample.

The invention further refers to a kit for assessing the risk of coronary heart disease, according to any one of methods in any of the preceding claims comprising: a) reagents for the detection of the metabolite markers as defined herein, b) a kit control, and c) optionally instructions for use.

### Experiments

### Study population and definition of endpoints

The study was conducted within the European multicentered BiomarCaRE project ("Biomarker for Cardiovascular Risk Assessment in Europe", http://www.biomarcare.eu/), (Zeller, Hughes et al. 2014), aiming at identifying biomarkers to improve risk estimation of cardiovascular diseases (CVD) in Europe. The baseline variables, phenotypes, event and follow-up information have been harmonized within the BiomarCaRE and the MORGAM (MONICA Risk, Genetics, Archiving and Monograph", http://www.thl.fi/morgam/) data base. For this study, a case-cohort set has been used, consisting of one cohort of all cases (i.e. all subjects of the individual cohorts developing any or all events of interest during the follow-up period) and another cohort of a random subsample of the original cohort, selected independently of the cases, so that included cases are possible. (Kulathinal, S., et al. (2007). "Case-cohort design in practice - experiences from the MORGAM Project." Epidemiol Perspect Innov 4: 15.)

For the BiomarCaRE case-cohort, data from six participating European cohort studies were used; namely FINRISK (Finland, n=2638), Brianza (Italy, n=674), Moli-Sani (Italy, n=2319), KORA (Germany, n=1583), RCPH (Glostrup, Denmark, n=4145), and SHHEC (UK, n=3698), in total comprising 15057 individuals.

Associations between metabolites and incident CHD events as the definite endpoint were analyzed. CHD was defined as first fatal or non-fatal CHD event during follow-up. Exclusion criteria were prevalent cardiovascular disease (CVD), prevalent heart failure (HF), and/or prevalent atrial fibrillation (AF).)

### Laboratory Analysis

The AbsoluteIDQ p180 Kit from Biocrates Life Sciences AG was used for sample preparation according to manufactures recommendation, quantifying up to 184 metabolites from five analytic groups (Amino acids, biogenic amines, Acylcarnitines, Glycerophospholipids, Sphingolipids and sugars). 10µl of serum per sample were prepared on a 96-well plate including 72 samples, 7 standards and 3 control samples (describe controls). For quantification, isotopelabeled internal standards and seven calibrators of different concentration levels were used. In addition, three different levels of quality controls were included (n = 2 QC1, n= 5 QC2, n= 2 QC3), from which QC 2 was used for data normalization, containing metabolites levels most comparable to physiological levels.

After preparation, samples were extracted with ammonium acetate/ MeOH (5mM) and were either diluted 1:2 in water (for Liquid chromatography mass spectrometry (LC-MS)) or 1:3.6 in flow injection analysis (FIA)-Solvent (for FIA-MS/MS). Measurements were performed on a TSQ Vantage (ThermoFisher Scientific) connected to an Ultimate 3000 Pump/ Autosampler (Dionex). Amino acids and biogenic amines were measured by LC-MS and were quantified in relation to isotope-labeled internal standards (e.g. Gly: 13C2-N15-gly; Arg: 15N2-Arg; Glu: d3-Glu; Gln: d5-Gln). Separation was done on a silica- based column (Syncronis aQ 50x2,1mm, 1.7µm), using an injection volume of 5µL. The mobile Phases were water with 0.5% formic acid (A) and acetonitrile with 0.5% formic acid (B) at a flow rate of 0.8 ml/min up to 1.0 ml/min. The gradient was 0-1.5 min A/B (%) 100/0; 1.5 - 2.7 min A/B (%) 82.5/17.5; 2.7 - 4 min A/B (%) 50/50; 4 - 5.25 min A/B (%) 5/95; 5.25 - 5.6 min A/B (%) 100/0. Metabolite identities were confirmed with the use of the internal standards. Raw data were processed with XCalibur Version 2.2 SP1.48 (Thermo Fisher Scientific) and MetIDQ (Biocrates Life Sciences AG). Acylcarnitines, glycerophospholipids, sphingolipids and sugars were injected directly into the mass spectrometer via flow injection without any previous separation. The injection volume was 10µL. Solvent was water with 3.4% FIA solvent (Biocrates Life Sciences AG) in methanol at a flow rate of 0.08 ml/min up to 0.2 ml/min. MS analyses were carried out with the use of electrospray ionization in positive- and negative-ion mode with a full scan. Other MS setting were: spray voltage 4000V (neg. mode) and 2800V (pos. mode), capillary temperature 300 °C, vaporizer temperature 120°C, sheath gas pressure 30 Arb, aux gas pressure 5 Arb, ion sweep gas pressure 0 arb, collision gas pressure 0.3 mTorr (neg. mode) and 1.0 mTorr (pos. mode), colliosion gas flow 0.15 mL/min, Q1 peak width 0.7 FWHM, cycle time 0.12 s (neg. mode) and 1.5 s in pos. mode), experimental type SRM.

### Statistical Analysis

### Data normalization

Normalization was performed across the entire BiomarCaRE case-cohort to ensure harmonization, which corrects each metabolite level by a factor determined from the ratio of the mean of all internal standards and the mean of the reference samples from the corresponding plate.

### Exclusion of metabolites and samples

Among valid metabolite measurements, some were incomplete, missing, or below the Limit of Detection (LOD). The Absolute***IDQ***® p180 Kit provides plate-specific LOD values for each metabolite for the FIA measurements, while the LOD values for the LC measurements were defined globally and independent of plates. In this study, a global LOD value was defined for each metabolite of the FIA part that corresponds to the highest LOD value across all plates being measured, while the LC LOD values were taken from the Biocrates p 180 Kit Manual.

Metabolites were excluded from all subsequent analyses when at least one of the following criteria was fulfilled: (1) > 30% of measurements were missing for FIA and LC part, (2) > 50% of measurements were below LOD, (3) > 50% of measurements were below LOD in a case-noncase comparison, or (4) > 30% of measurements were missing. Additionally, samples were excluded from the analyses if > 50% of metabolites were missing. As a consequence, all subsequent data analyses were performed on 141 metabolites and 13328 samples (FINRISK n=2208, Brianza n=583, Moli-Sani n=2169, KORA n=1451, RCPH Glostrup n=3880, and SHHEC n=2937) in total.

### Missing data imputation and data transformation

To impute single missing data points (i.e. missing measurements), the multiple imputation method *R: mice* was used to generate 10 complete datasets. The multivariate analysis was then performed for each imputed dataset separately, and the obtained estimates were combined into one pooled estimate.

Metabolite levels below LOD were imputed using a uniform distribution between LOD and LOD/2. As most metabolites showed skewed distributions, all data were log-transformed to approximate normal distribution (Grund, B. and C. Sabin (2010). "Analysis of biomarker data: logs, odds ratios, and receiver operating characteristic curves." Curr Opin HIV AIDS 5(6): 473-479.).

### Multivariate analysis

Associations with incident CHD events were assessed for each metabolite individually by applying Cox proportional Hazard models. The model was adjusted for the covariates age, sex, blood pressure, HDL and LDL cholesterol, body-mass-index (BMI), prevalent diabetes, the different cohort studies and medication (antihypertensive treatment, lipid lowering treatment). To assess the validity of the Proportional Hazards Assumption, a test for proportionality was performed for each covariate by generating graphs of scaled Schoenfeld residuals against time and by assessing their corresponding p-values. Here, evidence against proportionality was found for sex and smoking. Consequently, the model was stratified by sex and smoking. Additionally, to assess sex-specific differences, the analyses were repeated for men and women separately. This model served estimating 5-year risk of incident CHD, and subsequently 10-year and 15-year risk, too. For computing the 10-year risk, the Moli-Sani cohort study was excluded, while for the 15-year risk assessment, both Moli-Sani and FINRISK cohort studies were excluded as no long-term follow-up times were available.

Hazard ratios were reported per one SD and significance was met if p-values < 0.00035 (i.e. p-values < 0.05 after Bonferroni correction for multiple testing). All significant metabolites which obtained significant hazard ratios were assessed in subsequent analysis, including discrimination and reclassification.

### Discrimination and Reclassification

Discrimination of CHD risk was assessed by the C-statistic (Grund and Sabin 2010). To assess the utility of a metabolite serving as a clinical biomarker, the C-statistics for risk prediction models fitted were compared with and without the metabolite, while adjusting for the covariates age, sex, blood pressure, HDL and LDL cholesterol, body-mass-index (BMI), prevalent diabetes, the different cohort studies and medication (antihypertensive treatment, lipid lowering treatment). The baseline model was adjusted for these covariates only, while the models including individual metabolites were compared to the baseline based on their C-statistics. To avoid overfitting of the C-statistics, a 10-fold cross validation was used. Net reclassification improvement (NRI) was computed for all significant metabolites to assess their ability to reclassify individuals as compared to the baseline model.

### Metabolic signature

In order to identify a metabolite signature, which is the smallest set of metabolites that is able to predict CHD with maximal performance (Lagani, V., et al. (2013). "Biomarker signature identification in "omics" data with multi-class outcome." Comput Struct Biotechnol J 6:), we first applied a multivariate analysis for all possible combinations of significant metabolites, followed by assessing the C-statistic of each metabolite combination, revealing the combination with maximal predictive performance as the seeked metabolite signature. The predictive value of each metabolite signature was assessed by comparing their C-statistic values with the basic model and the C-statistic values of individual metabolites. To reduce the number of possible combinations of metabolites, their pair-wise correlation was assessed and, if two metabolites were highly correlated (Pearson correlation coefficient > 0.8), the metabolite which had the lower C-statistic was excluded from the signature search.

### Metabolite sums and ratios

The evaluation of individual metabolites was supported by the calculation of 35 predefined metabolite sums and ratios. Associations between these sums and ratios and incident CHD was determined using a multivariate analysis adjusting for covariates as previously described. These associations were determined for women, men, and both sexes combined (overall cohort), concordant with previous analyses.

### Results

### Baseline characteristics

Baseline characteristics of the study individuals are described in **Table 1.** Among the 13328 individuals investigated, our case-cohort set involved 5396 women (40.8%) and 7832 men (59.2%) with an average age of 56.4 years and 55.1 years, respectively. Among all individuals, 2166 (16,4%) experienced a CHD event. A higher proportion of men (18.7%) than women (13%) developed CHD. We observed significant differences between sexes in age, LDL/HDL-ratios, smoking habits, prevalence of diabetes, and antihypertensive medication intake (data not shown). To minimize the influence of these observations during the data analysis, the multivariate model was stratified for sex and smoking, while it was adjusted for the covariates study center, blood pressure (systolic BP), cholesterol (LDL/HDL ratio), body-mass-index, history of diabetes, antihypertensive and lipid lowering medication.

**Table1: Baseline Characteristics of the Study Population**

| | **Overall** | **Women** | **Men** |
|---|---|---|---|
| **Sex, No. (%)** | 13328 (100%) | 5396 (40.8%) | 7832 (59.2%) |
| **Age, mean (SD), yr** | 55.6 (11.6) | 56.4 (11.3) | 55.1 (11.7) |
| **Body-Mass-Index, mean (SD), kg/m2** | 27.3 (5.7) | 27.2 (6.2) | 27.3 (5.4) |
| **Systolic blood pressure, mean (SD), mm Hg** | 138.1 (21.8) | 137 (22.4) | 138.9 (21.2) |
| **Diastolic blood pressure, mean (SD), mm Hg** | 82.9 (11.4) | 81.1 (11.2) | 84.1 (11.5) |
| **Total cholesterol, median (SD), mg/dL** | 228.2 (46.3) | 235.9 (47.3) | 224.3 (45.2) |
| **HDL cholesterol, median (SD), mg/dL** | 53 (15.7) | 59.2 (16.3) | 49.1 (13.8) |
| **LDL cholesterol, median (SD), mg/dL** | 147 (37.7) | 147 (39.2) | 147 (36.6) |
| **LDL/HDL cholesterol ratio, median (SD), mg/dL** | 2.7 (1.1) | 2.5 (1.0) | 2.9 (1.1) |
| **Triglycerides, median (SD), mg/dL** | 132 (94.9) | 121 (77.5) | 141 (104) |
| **Anti-hypertensive medication, No. (%)** | 2533 (19.1%) | 1184 (21.9%) | 1349 (17.2%) |
| **Cholesterol - lowering medication, No. (%)** | 337 (2.5%) | 156 (1.2%) | 181 (1.4%) |
| **Smoking (daily), No. (%)** | 4139 (31.3%) | 1557 (28.9%) | 2582 (33.0%) |
| **History of diabetes mellitus, No. (%)** | 749 (5.7%) | 270 (5.0%) | 479 (6.1%) |
| **CHD total cases (fatal, non-fatal), No. (%)** | 2166 (16.4%) | 703 (13.0%) | 1463 (18.7%) |
| **CHD fatal cases, No. (%)** | 677 (5.1%) | 220 (4.1%) | 457 (5.8%) |
| **CHD non-fatal cases, No. (%)** | 1489 (11.3%) | 483 (9.0%) | 1006 (12.8%) |

### Individual metabolite associations with incident CHD

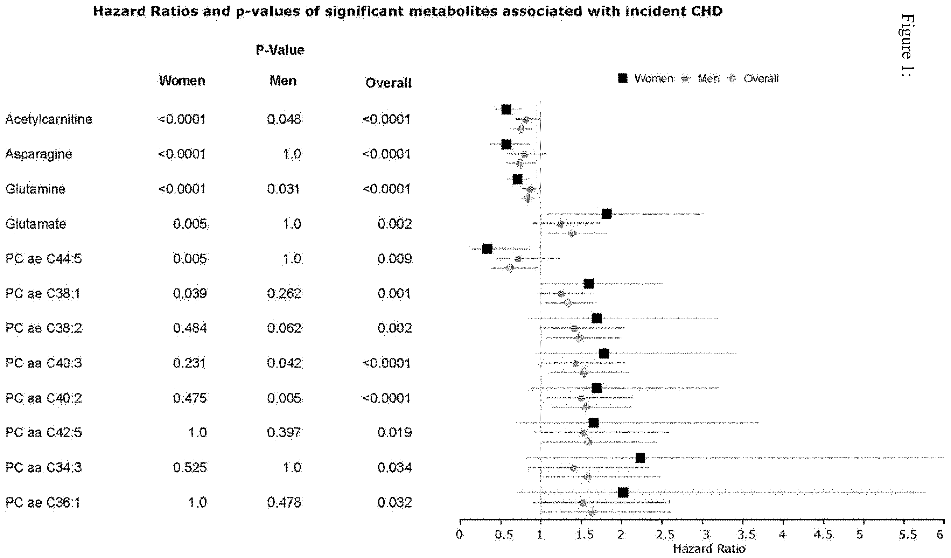

Associations of individual metabolites estimating 5-year risk of incident CHD events were assessed by applying Cox proportional Hazard models, adjusting for age, blood pressure, HDL and LDL cholesterol, body-mass-index (BMI), prevalent diabetes, the different cohort studies and medication, while the models were stratified by sex and smoking. Results showed 12 metabolites to be significantly associated with incident CHD, among which acetylcarnitine (C2) (HR=0.76; P-value=3,8·10⁻⁸), asparagine (Asn) (HR=0.74; P-value=4.6·10⁻⁴) and glutamine (Gln) (HR=0.84; P-value=1.3·10⁻⁷) were protective, while glutamate (Glu) (HR=1.38; P-value=2.2·10⁻³) was associated with high risk of CHD. Among the eight significant phospholipids, seven were observed with high risk for CHD (PC aa C34:3 (HR=1.58, P-value=3.4·10⁻²), PC ae C36:1 (HR=1.63, P-value=3.2·10⁻²), PC ae 38:1 (HR=1.33, P-value=1.1·10⁻³), PC ae 38:2 (HR=1.47, P-value=1.7·10⁻³), PC aa C40:2 (HR=1.55, P-value=6.4-10⁻⁵), PC aa C40:3 (HR=1.53, P-value=2.1·10⁻⁴), and PC aa C42:5 (HR=1.58, P-value=1.9·10⁻²)), while one phospholipid (PC ae C44:5) was protective (HR=0.61, P-value=9.1·10⁻³) **(****Figure 1****).**

Subsequently, men and women were analyzed separately, revealing a subset of these metabolites to be significant. In women, six metabolites were significantly associated with incident CHD **(****Figure 1****),** including acetylcarnitine (HR=0.57, P-value=1.9·10⁻¹⁰), asparagine (HR=0.57, P-value=3.1·10⁻⁴), glutamine (HR=0.71, P-value=2.2·10⁻⁷), glutamate (HR=1.81, P-value=4.7·10⁻³), and the two long-chained glycerophospholipids PC ae C38:1 (HR=1.59, P-value=3.9·10⁻²) and PC ae C44:5 (HR=0.33, P-value=4.9·10⁻³), while in men, four metabolites were identified **(****Figure 1****),** namely acetylcarnitine (HR=0.83, P-value=4.8·10⁻²), glutamine (HR=0.88, P-value=3.1·10⁻²) and the two long-chained glycerophospholipids PC aa C40:2 (HR=1.51, P-value=5.1·10⁻³) and PC aa C40:3 (HR=1.44, P-value=4.2·10⁻². The effect directions between both sexes were concordant with the overall results.

Our results revealed positive correlation, meaning possibly protective association, of C2, glutamine on CHD events in both sexes and of asparagine in women. Glutamate and in general all of the phospholipids showed a negative association of developing CHD in both sexes, except of PC ae C44:5.

### Discriminatory ability of metabolites in incident CHD

To assess the predictive utility of the identified metabolites in men and women, C-statistics of 5-year risk for each metabolite added to the basic model were computed. The results revealed three metabolites in women and one metabolite in men, showing an improved discriminatory ability as compared to the basic model. In women, C2 (0.8814; +0.0002), asparagine (0.8836; +0.0024), and the long-chained phospholipid PC ae C44:5 (0.8818; +0.0006) showed larger C-statistics than the basic model (0.8812), while the phospholipid PC aa C40:3 (0.8699; +0.0009) improved the prediction compared to the basic model (0.8869) in men. In both sexes combined, the results revealed seven metabolites with an improved discriminatory ability compared to the basic model (0.8846), namely, asparagine (0.8848, +0.0002) and phospholipids PC aa 34:3 (0.8848, +0.0002), PC ae 36:1 (0.8854, +0.0008), PC ae C38:1 (0.8849, +0.0003), PC aa C40:2 (0.8847, +0.0001), PC aa C40:3 (0.8852, +0.0006), and PC aa C42:5 (0.8849, +0.0003).

Net reclassification improvement (NRI) was computed for all significant metabolites to assess their ability to reclassify individuals as compared to the baseline model. Here, the individual metabolites showed no significant improvement.

### Discriminatory ability of metabolite signature in incident CHD

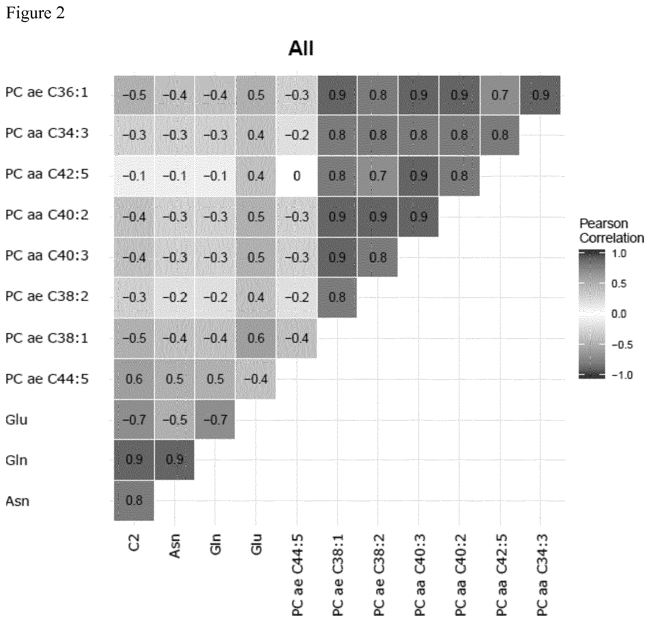

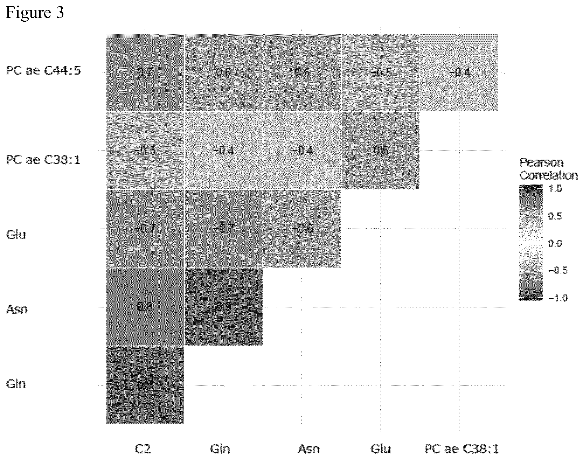

In order to identify a sex-specific and overall metabolite signature for incident CHD, the smallest set of metabolites was determined for each sex and across sexes, which showed the maximal discriminatory ability between individuals as compared to the basic model **(Table 4).** To minimize the number of metabolites in the signature, only significant metabolites were chosen. Among the significant phospholipids, only those were chosen that were not highly correlated with each other (Pearson Correlation Coefficient < 0.8) **(****Figures 2** **and** **3****).** Consequently, six phospholipids (PC aa 40:3, aa C34:3, ae C36:1, aa C42:5, ae C38:1, and ae C38:2) were excluded from the overall case-cohort signature computation.

Subsequently, metabolite signatures were determined for each sex separately and for both sexes combined by comparing the C-statistics of all possible metabolite combinations with the basic model and with models including the basic covariates and a single metabolite. In women, the combination of asparagine, C2 and PC ae C44:5 revealed the highest discriminatory ability (C-statistic = 0.8847 (+0.0035)), as compared to the basic model (C-statistic = 0.8812) **(Table 2)** and to their individual discriminatory abilities (asparagine: C-statistic = 0.8836; acetylcarnitine: C-statistic = 0.8814; PC ae C44:5: C-statistic = 0.8818). Across sexes, a metabolite signature was determined consisting of glutamine and asparagine (C-statistic = 0.8854 (+0.0008)), showing the best discriminatory ability as compared to the basic model (C-statistic = 0.8846).

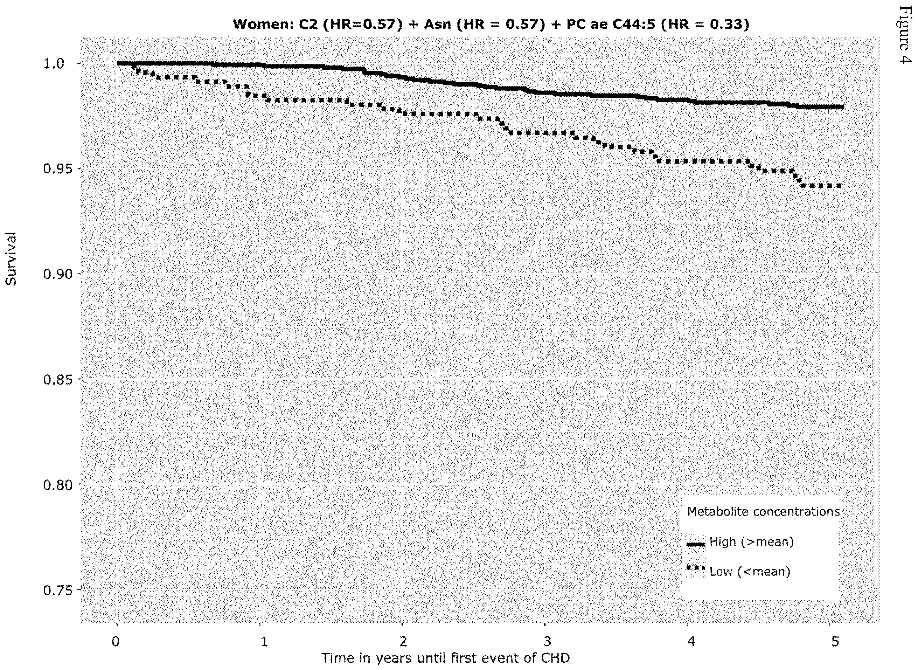

In order to document differences in event-free survival due to metabolite levels, survival curves were determined for low (< mean of metabolite measurements) and high (> mean of metabolite measurements) levels. **Figure 4** exemplarily illustrates a longer event-free time for high levels of the metabolite signature Asparagine, Acetylcarnitine and PC ae C44:5 in women.

**Table 2 -statistics of metabolite combinations in women. For all possible combinations of metabolites that are significantly associated with incident CHD in women, the C-statistics based on a 10-fold cross validation are presented. The baseline model was adjusted for studies, blood pressure (systolic BP), cholesterol (LDL/HDL ratio), body-mass-index, history of diabetes, and medication (antihypertensive treatment, lipid treatment), and stratified for smoking. For each significant metabolite combination the C-Index, 95% confidence interval, and its difference between its C-Index and the baseline's C-Index are presented. Positive differences are shown in italic letters.**

| | **C**-**Statistic** | **95% Confidence Interval** | **Difference** |
|---|---|---|---|
| **Baseline** | **0.8812** | **[0.8440;0.9184]** | |
| | | | |
| ***C2*+ *ASPARAGINE*** | ***0.8841*** | ***[0.8469;0.9213]*** | ***0.0029*** |
| C2+ GLUTAMINE | 0.8810 | [0.8438;0.9182] | -0.0002 |
| C2+ GLUTAMATE | 0.8806 | [0.8434;0.9178] | -0.0006 |
| C2+ PC AE C38:1 | 0.8807 | [0.8436;0.9179] | -0.0005 |
| *C2*+ *PC C44:5* | *0.8816* | *[0.8444;0.9188]* | *0.0004* |
| *ASPARAGINE*+ *GLUTAMINE* | *0.8832* | *[0.8460;0.9204]* | *0.0020* |
| *ASPARAGINE*+ *GLUTAMATE* | *0.8823* | *[0.8451;0.9195]* | *0.0011* |
| *ASPARAGINE*+ *PC AE C38:1* | *0.8824* | *[0.8452;0.9196]* | *0.0012* |
| ***ASPARAGINE*+ *PC AE C44:5*** | ***0.8842*** | ***[0.8470;0.9214]*** | ***0.0030*** |
| GLUTAMINE+ GLUTAMATE | 0.8798 | [0.8426;0.9170] | -0.0014 |
| GLUTAMINE+ PC AE C38:1 | 0.8798 | [0.8426;0.9170] | -0.0014 |
| *GLUTAMINE*+ *PC AE C44:5* | *0.8815* | *[0.8444;0.9187]* | *0.0003* |
| GLUTAMATE + PC AE C38:1 | 0.8790 | [0.8419;0.9162] | -0.0022 |
| GLUTAMATE + PC AE C44:5 | 0.8810 | [0.8439;0.9182] | -0.0001 |
| PC AE C38:1+ PC AE C44:5 | 0.8807 | [0.8436;0.9179] | -0.0005 |
| *C2*+ *ASPARAGINE*+ *GLUTAMINE* | *0.8838* | *[0.8466;0.9209]* | *0.0026* |
| *C2*+ *ASPARAGINE*+ *GLUTAMATE* | *0.8831* | *[0.8460;0.9203]* | *0.0019* |
| *C2*+ *ASPARAGINE*+ *PC AE C38:1* | *0.8834* | *[0.8463;0.9206]* | *0. 0023* |
| ***C2*+ *ASPARAGINE***+ ***PC AE C44:5*** | ***0.8847*** | ***[0.8476;0.9219]*** | ***0.0035*** |
| C2+ GLUTAMINE+ GLUTAMATE | 0.8802 | [0.8430;0.9174] | -0.0010 |
| C2+ GLUTAMINE+ PC AE C38:1 | 0.8806 | [0.8434;0.9177] | -0.0006 |
| *C2*+ *GLUTAMINE*+ *PC AE C44:5* | *0.8813* | *[0.8441;0.9184]* | *0.0001* |
| C2+ GLUTAMATE + PC AE C38:1 | 0.8797 | [0.8425;0.9169] | -0.0015 |
| C2+ GLUTAMATE + PC AE C44:5 | 0.8810 | [0.8438;0.9182] | -0.0002 |
| C2+ PC AE C38:1+ PC AE C44:5 | 0.8808 | [0.8437;0.9180] | -0.0004 |
| *ASPARAGINE*+ *GLUTAMINE*+ *GLUTAMATE* | *0.8820* | *[0.8449;0.9192]* | *0.0008* |
| *ASPARAGINE*+ *GLUTAMINE*+ *PC AE C38:1* | *0.8821* | *[0.8449;0.9193]* | *0. 0009* |
| *ASPARAGINE*+ *GLUTAMINE*+ *PC AE C44:5* | *0.8836* | *[0.8464;0.9208]* | *0.0024* |
| ASPARAGINE+ GLUTAMATE + PC AE C38:1 | 0.8811 | [0.8439;0.9183] | -0.0001 |
| *ASPARAGINE*+ *GLUTAMATE* + *PCAE C44:5* | *0.8834* | *[0.8462;0.9205]* | *0.0022* |
| *ASPARAGINE*+ *PC AE C38:1*+ *PC AE C44:5* | *0.8832* | *[0.8460;0.9204]* | *0.0020* |
| GLUTAMINE+ GLUTAMATE + PC AE C38:1 | 0.8787 | [0.8415;0.9158] | -0.0025 |
| GLUTAMINE+ GLUTAMATE + PC AE C44:5 | 0.8808 | [0.8436;0.9180] | -0.0004 |
| GLUTAMINE+ PC AE C38:1+ PC AE C44:5 | 0.8803 | [0.8432;0.9175] | -0.0009 |
| GLUTAMATE + PC AE C38:1+ PC AE C44:5 | 0.8796 | [0.8424;0.9168] | -0.0016 |
| | | | |
| *C2*+ *ASPARAGINE*+ *GLUTAMINE*+ *GLUTAMATE* | *0.8829* | *[0.8457;0.9201]* | *0.0017* |
| *C2*+ *ASPARAGINE*+ *GLUTAMINE*+ *PC AE C38:1* | *0.8832* | *[0.8460;0.9204]* | *0.0020* |
| *C2*+ *ASPARAGINE*+ *GLUTAMINE*+ *PC AE C44:5* | *0.8842* | *[0.8470;0.9214]* | *0. 0030* |
| *C2*+ *ASPARAGINE*+ *GLUTAMATE*+ *PC AE C38:1* | *0.8823* | *[0.8451;0.9194]* | *0.0011* |
| *C2*+ *ASPARAGINE*+ *GLUTAMATE*+ *PC AE C44:5* | *0.8839* | *[0.8467;0.9211]* | *0. 0027* |
| *C2*+ *ASPARAGINE*+ *PC AE C38:1*+ *PC AE C44:5* | *0.8840* | *[0.8469;0.9212]* | *0.0028* |
| C2+ GLUTAMINE+ GLUTAMATE + PC AE C38:1 | 0.8795 | [0.8423;0.9167] | -0.0017 |
| C2+ GLUTAMINE+ GLUTAMATE + PC AE C44:5 | 0.8807 | [0.8436;0.9179] | -0.0005 |
| C2+ GLUTAMINE+ PC AE C38:1+ PC AE C44:5 | 0.8809 | [0.8437;0.9181] | -0.0003 |
| C2+ GLUTAMATE + PC AE C38:1+ PC AE C44:5 | 0.8797 | [0.8425;0.9168] | -0.0015 |
| ASPARAGINE+ GLUTAMINE+ GLUTAMATE + PC AE C38:1 | 0.8805 | [0.8434;0.9177] | -0.0007 |
| *ASPARAGINE*+ *GLUTAMINE*+ *GLUTAMATE*+ *PC AE C44:5* | *0.8832* | *[0.8460;0.9203]* | *0.0020* |
| *ASPARAGINE*+ *GLUTAMINE*+ *PC AE C38:1* + *PC AE C44:5* | *0.8829* | *[0.8457;0.9201]* | *0.0017* |
| *ASPARAGINE*+ *GLUTAMATE* + *PC AE C38:1* + *PC AE C44:5* | *0.8826* | *[0.8454;0.9198]* | *0.0014* |
| GLUTAMINE+ GLUTAMATE + PC AE C38:1+ PC AE C44:5 | 0.8793 | [0.8421;0.9165] | -0.0019 |
| | | | |
| *C2*+ *ASPARAGINE*+ *GLUTAMINE*+ *GLUTAMATE* + *PC AE C38:1* | *0.8818* | *[0.8446;0.9189]* | *0.0006* |
| *C2*+ *ASPARAGINE*+ *GLUTAMINE*+ *GLUTAMATE* + *PC AE C44:5* | *0.8834* | *[0.8462;0.9206]* | *0.0022* |
| *C2+ ASPARAGINE*+ *GLUTAMINE*+ *PC AE C38:1* + *PC AE C44:5* | *0.8836* | *[0.8464;0.9208]* | *0.0024* |
| *C2+ ASPARAGINE+ GLUTAMATE + PC AE C38:1* + *PC AE C44:5* | *0.8828* | *[0.8456;0.9200]* | *0.0016* |
| C2+ GLUTAMINE+ GLUTAMATE + PC AE C38:1+ PC AE C44:5 | 0.8795 | [0.8424;0.9167] | -0.0017 |
| *ASPARAGINE*+ *GLUTAMINE*+ *GLUTAMATE* + *PC AE C38:1* + *PC AE C44: 5* | *0.8823* | *[0.8451;0.9195]* | *0.0011* |
| | | | |
| *C2*+ *ASPARAGINE*+ *GLUTAMINE*+ *GLUTAMATE* + *PC AE C38:1* + *PC AE C44:5* | *0.8824* | *[0.8452;0.9196]* | *0.0012* |

**Table 3 C-statistics of metabolite combinations in the overall case cohort. For all possible combinations of metabolites that are significantly associated with incident CHD in the entire case cohort, the C-statistics based on a 10-fold crossvalidation are presented. The baseline model was adjusted for European cohort studies, blood pressure (systolic BP), cholesterol (LDL/HDL ratio), body-mass-index, history of diabetes, and medication (antihypertensive treatment. lipid treatment), and stratified for smoking. For each significant metabolite combination the C-Index, 95% confidence interval, and its difference between its C-Index and the baseline's C-Index are presented. Positive differences are shown in italic letters.**

| | **C**-**Statistic** | **95% Confidence Interval** | **Difference** |
|---|---|---|---|
| **Baseline** | **0.8846** | **[0.8644;0.9048]** | |
| | | | |
| C2 + GLUTAMINE | 0.8840 | [0.8638;0.9042] | -0.0006 |
| C2 + ASPARAGINE | 0.8846 | [0.8644;0.9048] | 0.0000 |
| C2 + GLUTAMATE | 0.8831 | [0.863;0.9033] | -0.0015 |
| C2 + PC AA C40:2 | 0.8834 | [0.8632;0.9036] | -0.0012 |
| C2 + PC AA C44:5 | 0.8833 | [0.8631;0.9035] | -0.0013 |
| ***GLUTAMINE*+ *ASPARAGINE*** | ***0.8854*** | ***[0.8652;0.9056]*** | ***0***.***0008*** |
| GLUTAMINE+ GLUTAMATE | 0.8844 | [0.8642;0.9046] | -0.0002 |
| GLUTAMINE+ PC AA C40:2 | 0.8846 | [0.8644;0.9048] | 0.0000 |
| GLUTAMINE+ PC AA C44:5 | 0.8845 | [0.8643;0.9047] | -0.0001 |
| *ASPARAGINE*+ *GLUTAMATE* | *0.8852* | *[0.865;0.9053]* | *0.0006* |
| *ASPARAGINE*+ *PC AA C44:2* | *0.8853* | *[0.8651;0.9054]* | *0.0006* |
| *ASPARAGINE*+ *PC AA C44:5* | *0.8852* | *[0.865;0.9054]* | *0.0006* |
| GLUTAMATE+ PC AA C40:2 | 0.8837 | [0.8635;0.9039] | -0.0009 |
| GLUTAMATE+ PC AA C44:5 | 0.8835 | [0.8633;0.9037] | -0.0011 |
| PC AA C40:2+ PC AA C44:5 | 0.8840 | [0.8638;0.9042] | -0.0006 |
| | | | |
| C2 + GLUTAMINE+ ASPARAGINE | 0.8845 | [0.8643;0.9047] | -0.0001 |
| C2 + GLUTAMINE+ GLUTAMATE | 0.8835 | [0.8633;0.9037] | -0.0011 |
| C2 + GLUTAMINE+ PC AA C40:2 | 0.8837 | [0.8635;0.9039] | -0.0009 |
| C2 + GLUTAMINE+ PC AA C44:5 | 0.8836 | [0.8634;0.9038] | -0.0010 |
| C2 + ASPARAGINE+ GLUTAMATE | 0.8843 | [0.8642;0.9045] | -0.0003 |
| C2 + ASPARAGINE+ PC AA C40:2 | 0.8843 | [0.8641;0.9045] | -0.0003 |
| C2 + ASPARAGINE+ PC AA C44:5 | 0.8843 | [0.8641;0.9045] | -0.0003 |
| C2 + GLUTAMATE+ PC AA C40:2 | 0.8828 | [0.8626;0.903] | -0.0018 |
| C2 + GLUTAMATE+ PC AA C44:5 | 0.8828 | [0.8626;0.903] | -0.0018 |
| C2 + PC AA C40:2+ PC AA C44:5 | 0.8831 | [0.8629;0.9033] | -0.0015 |
| *GLUTAMINE*+ *ASPARAGINE*+ *GLUTAMATE* | *0.8851* | *[0.8649;0.9053]* | *0.0005* |
| *GLUTAMINE*+ *ASPARAGINE*+ *PC AA C40:2* | *0.8851* | *[0.8649;0.9053]* | *0.0005* |
| *GLUTAMINE*+ *ASPARAGINE*+ *PC AA C44:5* | *0.8851* | *[0.8649;0.9053]* | *0.0005* |
| GLUTAMINE+ GLUTAMATE+ PC AA C40:2 | 0.8841 | [0.8639;0.9043] | -0.0005 |
| GLUTAMINE+ GLUTAMATE+ PC AA C44:5 | 0.8840 | [0.8639;0.9042] | -0.0006 |
| GLUTAMINE+ PC AA C40:2+ PC AA C44:5 | 0.8843 | [0.8641;0.9045] | -0.0003 |
| *ASPARAGINE*+ *GLUTAMATE*+ *PC AA C40:2* | *0.8849* | *[0.8647;0.905]* | *0.0002* |
| *ASPARAGINE*+ *GLUTAMATE*+ *PCAAC44:5* | *0.8848* | *[0.8646;0.905]* | *0.0002* |
| *ASPARAGINE*+ *PC AA C40:2*+ *PC AA C44:5* | *0.8849* | *[0.8647;0.9051]* | *0.0003* |
| GLUTAMATE+ PC AA C40:2+ PC AA C44:5 | 0.8833 | [0.8631;0.9035] | -0.0013 |
| | | | |
| C2 + GLUTAMINE+ ASPARAGINE+ GLUTAMATE | 0.8842 | [0.864;0.9044] | -0.0004 |
| C2 + GLUTAMINE+ ASPARAGINE+ PC AA C40:2 | 0.8842 | [0.8641;0.9044] | -0.0004 |
| C2 + GLUTAMINE+ ASPARAGINE+ PC AA C44:5 | 0.8842 | [0.864;0.9044] | -0.0004 |
| C2 + GLUTAMINE+ GLUTAMATE+ PC AA C40:2 | 0.8832 | [0.863;0.9034] | -0.0014 |
| C2 + GLUTAMINE+ GLUTAMATE+ PC AA C44:5 | 0.8832 | [0.863;0.9034] | -0.0014 |
| C2 + GLUTAMINE+ PC AA C40:2+ PC AA C44:5 | 0.8834 | [0.8632;0.9036] | -0.0012 |
| C2 + ASPARAGINE+ GLUTAMATE+ PC AA C40:2 | 0.8839 | [0.8637;0.9041] | -0.0007 |
| C2 + ASPARAGINE+ GLUTAMATE+ PC AA C44:5 | 0.8841 | [0.8639;0.9042] | -0.0006 |
| C2 + ASPARAGINE+ PC AA C40:2+ PC AA C44:5 | 0.8840 | [0.8638;0.9042] | -0.0006 |
| C2 + GLUTAMATE+ PC AA C40:2+ PC AA C44:5 | 0.8824 | [0.8622;0.9026] | -0.0022 |
| *GLUTAMINE*+ *ASPARAGINE*+ *GLUTAMATE*+ *PC AA C40:2* | *0.8848* | *[0.8646;0.905]* | *0.0002* |
| *GLUTAMINE*+ *ASPARAGINE*+ *GLUTAMATE*+ *PC AA C44:5* | *0.8847* | *[0.8646;0.9049]* | *0.0001* |
| *GLUTAMINE*+ *ASPARAGINE*+ *PC AA C40: 2*+ *PC AA C44:5* | *0.8848* | *[0.8646;0.905]* | *0.0002* |
| GLUTAMINE+ GLUTAMATE+ PC AA C40:2+ PC AA C44:5 | 0.8838 | [0.8636;0.904] | -0.0008 |
| ASPARAGINE+ GLUTAMATE+ PC AA C40:2+ PC AA C44:5 | 0.8845 | [0.8643;0.9047] | -0.0001 |
| | | | |
| C2 + GLUTAMINE+ ASPARAGINE+ GLUTAMATE+ PC AA C40:2 | 0.8838 | [0.8636;0.904] | -0.0008 |
| C2 + GLUTAMINE+ ASPARAGINE+ GLUTAMATE+ PC AA C44:5 | 0.8840 | [0.8638;0.9042] | -0.0006 |
| C2 + GLUTAMINE+ ASPARAGINE+ PC AA C40:2+ PC AA C44:5 | 0.8839 | [0.8637;0.9041] | -0.0007 |
| C2 + GLUTAMINE+ GLUTAMATE+ PC AA C40:2+ PC AA C44:5 | 0.8829 | [0.8627;0.9031] | -0.0017 |
| C2 + ASPARAGINE+ GLUTAMATE+ PC AA C40:2+ PC AA C44:5 | 0.8836 | [0.8634;0.9038] | -0.0010 |
| GLUTAMINE+ ASPARAGINE+ GLUTAMATE+ PC AA C40:2+ PC AA C44:5 | 0.8845 | [0.8643;0.9047] | -0.0001 |
| | | | |
| C2 + GLUTAMINE+ ASPARAGINE+ GLUTAMATE+ PC AA C40:2+ PC AA C44:5 | 0.8835 | [0.8633;0.9037] | -0.0011 |

**Table 4: Discrimination (C-statistic) for metabolites significantly associated with incident CHD after a 10-fold cross validation. The baseline model was adjusted for cohort, blood pressure (systolic BP), cholesterol (LDL/HDL ratio), body-mass-index, history of diabetes, and medication (antihypertensive treatment, lipid lowering treatment), and stratified for smoking. For each significant metabolite the C-Statistic, 95% confidence interval (CI), and its difference between its C-statistic and the baseline's C-statistic are presented. Positive differences are shown in italic. No differences were significant.**

| | **Women** | | **Men** | | **Overall** | |
|---|---|---|---|---|---|---|
| | *C-Statistic (CI)* | *Difference* | *C- Statistic (CI)* | *Difference* | *C- Statistic (CI)* | *Difference* |
| **Baseline** | **0.8812 [0.8440;0.9184]** | | **0.8690 [0.8448;0.8933]** | | **0.8846 [0.8644;0.9048]** | |
| **C2** | 0.8814 [0.8442;0.9185] | *0.0002* | 0.8680 [0.8438;0.8922] | -0.0010 | 0.8837 [0.8635;0.9039] | -0.0009 |
| **Asn** | 0.8836 [0.8464;0.9207] | *0.0024* | | | 0.8848 [0.8646;0.9050] | *0.0002* |
| **Gln** | 0.8804 [0.8432;0.9176] | -0.0008 | 0.8690 [0.8449;0.8933] | 0.0000 | 0.8845 [0.8643;0.9047] | -0.0001 |
| **Glu** | 0.8804 [0.8432;0.9176] | -0.0008 | | | 0.8842 [0.8640;0.9044] | -0.0004 |
| **PC aa C34:3** | | | | | 0.8848 [0.8646;0.9050] | *0.0002* |
| **PC ae C36:1** | | | | | 0.8854 [0.8652;0.9056] | *0.0008* |
| **PC ae C38:1** | 0.8803 [0.8431;0.9175] | -0.0009 | | | 0.8849 [0.8647;0.9051] | *0.0003* |
| **PC ae C38:2** | | | | | 0.8843 [0.8641;0.9045] | -0.0003 |
| **PC aa C40:2** | | | 0.8685 [0.8443;0.8927] | -0.0005 | 0.8847 [0.8645;0.9049] | *0.0001* |
| **PC aa C40:3** | | | 0.8699 [0.8457;0.8941] | *0.0009* | 0.8852 [0.8650;0.9054] | *0.0006* |
| **PC aa C42:5** | | | | | 0.8849 [0.8647;0.9051] | *0.0003* |
| **PC ae C44:5** | 0.8818 [0.8446;0.9189] | *0.0006* | | | 0.8842 [0.8640;0.9044] | -0.0004 |

**Table 5: Concentration measurements of metabolites significantly associated with incident CHD. For all significant metabolites the mean levels (in µM) and their corresponding 95% confidence intervals (CI) are given for women, men, and the entire case-cohort (overall).**

| | **Women** | **Men** | **Overall** |
|---|---|---|---|
| | *Concentration measurement (CI)* | *Concentration measurement (CI)* | *Concentration measurement (CI)* |
| **C2** | 1.62 [0.83;2.34] | 1.51 [0.67;2.22] | 1.57 [0.75;2.30] |
| **Asn** | 6.45 [5.90;6.92] | 6.39 [5.52;6.94] | 6.42 [5.81;6.93] |
| **sGln** | 1.33 [0.47;1.94] | 1.27 [0.44;1.92] | 1.30 [0.44;1.94] |
| **Glu** | 1.55 [0.97;2.07] | 1.52 [0.97;2.03] | 1.54 [0.97;2.05] |
| **PC aa C34:3** | 3.94 [3.45;4.38] | 3.92 [3.46;4.42] | 3.93 [3.46;4.41] |
| **PC ae C36:1** | 2.83 [1.63;3.78] | 2.73 [1.65;3.63] | 2.78 [1.64;3.72] |
| **PC ae C38:1** | 2.84 [1.24;3.63] | 2.72 [1.14;3.59] | 2.79 [1.18;3.62] |
| **PC ae C38:2** | 5.49 [4.93;6.17] | 5.72 [5.14;6.33] | 5.59 [5.00;6.26] |
| **PC aa C40:2** | 0.33 [0.03;0.77] | 0.32 [0.03;0.76] | 0.33 [0.03;0.76] |
| **PC aa C40:3** | 0.27 [0.03;0.63] | 0.24 [0.01;0.62] | 0.26 [0.02;0.63] |
| **PC aa C42:5** | 3.61 [2.68;4.23] | 3.56 [2.65;4.25] | 3.59 [2.67;4.24] |

## Claims

1. Use of a metabolite marker combination comprising at least two metabolite markers for assessing the risk of coronary heart disease in a subject, wherein one metabolite marker is asparagine and wherein at least one further metabolite marker is selected from the group consisting of glutamine, glutamate, acetylcarnitine and phosphoglyceride.

2. Use of claim 1 wherein the phosphoglyceride is selected from the group consisting of PC aa C40:2, PC ae C44:5, PC aa C43:3 and PC ae C38:1.

3. Use of any one of the preceding claims, wherein the metabolite marker combination comprising at least two metabolite markers is selected from the group consisting of:
- asparagine and glutamine,
- asparagine and acetylcarnitine,
- asparagine, acetylcarnitine and PC ae C44:5,
- asparagine and PC aa C40:2,
- asparagine and PC ae C44:5,
- asparagine, glutamine and glutamate,
- asparagine, glutamine and PC aa C40:2,
- asparagine, glutamine and PC ae C44:5,
- asparagine, glutamate and PC aa C40:2,
- asparagine, glutamate and PC ae C44:5,
- asparagine, PC aa C40:2 and PC ae C44:5,
- asparagine, glutamine, glutamate and PC aa C40:2,
- asparagine, glutamine, glutamate and PC ae C44:5,
- asparagine, glutamine, PC aa C40:2 and PC ae C44:5, and
- asparagine, glutamate, PC aa C34:3 and PC ae C44:5.

4. Use of any one of the preceding claims, wherein the metabolite marker combination comprising at least two metabolite markers is selected from the group consisting of:
- asparagine and glutamine,
- asparagine and acetylcarnitine,
- asparagine and PC ae C44:5,
- asparagine, acetylcarnitine and PC ae C44:5, and
- asparagine, glutamate, PC aa C34:3 and PC ae C44:5.

5. Use of any one of the preceding claims, wherein the metabolite marker combination comprising at least two metabolite markers is selected from the group consisting of:
- asparagine and glutamine, and
- asparagine, glutamate, PC aa C34:3 and PC ae C44:5.

6. Use of any one of the preceding claims, wherein the metabolite marker combination comprising at least two metabolite markers is selected from the group consisting of:
- asparagine and acetylcarnitine, and
- asparagine, acetylcarnitine and PC ae C44:5.

7. Use of any one of the preceding claims, wherein the subject is male or female.

8. Use of any one of the preceding claims, wherein the subject is female.

9. Use of any one of the preceding claims, wherein the subject did not suffer from a cardiovascular event.

10. Use of any one of the preceding claims, wherein the subject did suffer from a cardiovascular event.

11. Use of any one of the preceding claims, wherein the sample is a body fluid of the subject, preferably a urine sample of the subject, more preferably a blood sample of the subject.

12. Use of any one of the preceding claims, wherein the level of the maker is detected by mass spectrometry or nuclear magnetic resonance spectroscopy.

13. A method for assessing the risk of coronary heart disease in a subject comprising the following steps:
a) detecting the level of at least two makers in a sample from the subject, wherein the at least two metabolite markers are defined in claim 1 to 6;
b) assessing the risk of coronary heart disease in the subject based on the level of the at least two makers.

14. Method according to claim 13, wherein step b) comprises comparing the level of the metabolite markers in the sample to a standard, wherein the level of the metabolite markers in the sample of the subject is indicative for the risk of coronary heart disease in the subject.

15. Method for assessing the risk of coronary heart disease in a subject comprising the following steps:
a) providing the sample to a laboratory for detecting the level of at least two makers in a sample, wherein the at least two metabolite markers are defined in claim 1 to 6;
b) assessing the risk of coronary heart disease in the subject based on the level of the at least two metabolite makers based on the report provided from the laboratory indicating the level of the at least two markers in the sample.
